# EUROPEAN PATENT SPECIFICATION

(11) **EP 1 674 051 B1**
(45) Date of publication and mention of the grant of the patent: **15.08.2007**
(21) Application number: 04030665.6
(22) Date of filing: 23.12.2004
(51) Int. Cl.: A61F 2/30

(54) **A method of surface finishing a bone implant**
Verfahren zum Oberfläche-Fertigbearbeiten von Knochenimplantaten
Procédé de finissage de surfaces d'implants osseux

(43) Date of publication of application: 28.06.2006
(73) Proprietor: Plus Orthopedics AG, 6343 Rotkreuz (CH)
(72) Inventor: Zinger, Oliver, 5032 Rohr AG (CH); Schmotzer, Hans, 8048 Zürich-Altstetten (CH)
(74) Representative: Popp, Eugen

(56) References cited:
- EP-A- 0 987 031
- EP-A- 1 386 591
- EP-A- 1 459 845
- WO-A-20/04008984
- US-A- 5 344 494
- US-A- 6 025 536
- US-A1- 2002 143 404

## Description

The present invention relates to a method of surface finishing a bone implant for particular use in orthopaedic or dental procedure, to which the closest prior art is WO 2004/008984A.

Such an implant is preferably manufactured so that in use it becomes integrated into the bone tissue. The implant must also be made of a material that is non-corrosive and that does not produce any immunological reaction causing rejection by the body. Typically, therefore such implants are metallic in nature and usually made from titanium, zirconium, niobium, or tantalum, or an alloy based on any of the aforesaid elements; medical grade stainless steel, or a cobalt-chromium alloy could also be used.

Events that lead to the integration of an implant into bone, and hence that determine the long-term performance of the implant, take place largely at the interface formed between the tissue and the implant. The development of this interface is complex and is influenced by numerous factors, including surface chemistry, surface charge, surface topography and surface contamination of the implant. To improve bone tissue integration, various techniques have been used to increase the surface roughness of titanium implants, and include: machining/micromachining, particle blasting, titanium plasma-spraying, chemical/electrochemical etching, particle blasting and chemical etching, electrochemical anodization, and pulsed laser ablation. Among these methods, abrasive blasting, also called sand blasting or grit blasting, is one of the most commonly found for metallic implants having surfaces that come into contact with bone that is to intergrow therewith. Implants of this kind are used as prostheses in orthopaedics, for replacing broken or diseased bone, and in dentistry, for building artificial teeth. Nowadays, abrasive blasting is widely used to produce micro-rough surfaces on implantable devices, with typically 4-6 micrometers of Ra (average roughness, according to ISO 4287-1997 and ASME 846.1-1995). This process is widely used because it is efficient for roughening a surface while being cost effective and shows excellent clinical results. In a histological study of well-fixed grit-blasted Ti-6Al-7Nb stems of hip replacements, [A. Zweymüller, F. K. Lintner and M. F. Semlitsch, Journal of Clinical Orthopedics 235,195-206 (1988)], it was found that excellent osseo-integration had been obtained as a result of the micro-roughness of the implant surfaces. It has also been found that osseo-integration occurs even under osteoporotic conditions: a grit-blasted Ti-6Al-7Nb prosthesis retrieved from a 100-year-old patient 3 years after arthroplasty showed sound bony fixation [D. K. Lester and P. Campbell, American Journal of Orthopaedics 25(1), 30-34 (1996)*].* It has also been reported that an examination of 17 acetabular components that had been in situ for between 10 days and 2.5 months found that, during the insertion of the treated socket, the rough cp titanium surface becomes covered with a slurry of bone particles and blood plasma. This mixture of living cells, inorganic substances, and native bone-growth promoting agents (BMPs, growth factors, etc.) adheres to the surface and allows osteogenesis to occur, regardless of whether or not the implant is in direct contact with the host bone, [F. K. Lintner, M. Huber, G. Böhm, J. Attems and R. Wais, in 15 Jahre Zweymüller-Hüftendoprothese, III. Wiener Symposium, K. Zweymüller, Eds. (Verlag Hans Huber, Bern, 1996) pp. 131-137*].* It has been reported by Delaunay & Kapandji [C. P. Delaunay and A. I. Kapandji, Journal of Arthroplasty 11(6), 643-652 (1996)] that they obtained a cumulative 6-7 year survival rate of approximately 98% when using a "Zweymüller stem". Extended clinical follow-up by these authors also found that grit-blasted cp titanium threaded cups to have a 9-10 year survival rate of approximately 99% [C. P. Delaunay and A. I. Kapandji, Journal of Clinical Orthopaedics 340, 130-141 (1997)*;* and C. P. Delaunay and A. I. Kapandji, Acta Orthop Scand 69(4),379-383 (1998)].

US Patent 5,456,723 discloses a method of producing such a metallic bone implant with a micro-roughness of 2 µm or less by subjecting the surface of the implant to pickling in a reducing acid. Such a micro-roughness may be applied directly to the surface of the implant by the pickling process or be super-imposed upon a micro-roughness having an Rt (peak to valley height, according to ISO 4287-1997 and ASME B46.1-1995) in excess of 10 µm produced by sand-blasting. In the latter case, the pickling step is so long and aggressive that the amount of the base material removed enables direct release and detachment of all the particles left behind by the sand-blasting, while producing an additional porous micro-topography smaller than 2 µm.

Many studies have analyzed the wear particles generated in joint replacement articulations and their effects on periprosthetic tissues. Additional third-body wear has been reported in cobalt-based alloy bearing surfaces for total hip replacement (THR) endoprotheses, originating for example from radio-pacifying agents in bone cements and from hydroxyapatite coatings [M. Rokkum, M. Brandt, K. Bye et al., The Journal of Bone and Joint Surgery 81-B(4), 582-589 (1999*)*]. Extensive surface inclusions from silicon- and/or aluminium-containing materials were found when five retrieved cementless implants with grit blasted surfaces were analyzed [J. L. Ricci, F. J. Kummer, H. Alexander and R. S. Casar, Journal of Applied Biomaterials 3, 225-230 (1992)]*.* Evidence of the same contaminants was found in soft-tissue samples adjacent to these implants, and also in a variety of new, non-implanted devices from different manufacturers. While in one experimental study alumina-blasting particles were considered to be a possible cause of tissue breakdown [B. W. Darvell, N. Samman, W. K. Luk, R.K.F. Clark and H. Tideman, Journal of Dentistry 23(5), 319-322 (1995)]*,* in other experimental studies these surface contaminants on blasted titanium implants seemed not to have any negative effects on the rate of bone on-growth [V. M. Goldberg, S. Stevenson, J. Feighan and D. Davy, Clinical Orthopaedics 319, 122-129 (1995)*;* and A. Piattelli, L. Manzon, A. Scarano, M. Paolantonio and M. Piattelli, International Journal of Oral & Maxillofacial Implants 13, 805-810 (1998)*].* More recently, Plenk et al. [H. Plenk Jr, M. Boehler, I. Steffan and A. Walter, European Cells and Materials 7(1), 78 (2004*)*]] showed that particle impaction of blasted implant surfaces leads to contamination of peri-implant tissues and metallic wear, but seems not directly to hinder bony anchorage. However, alumina particles produce increased third-body wear on both, the low and high carbon cobalt-based alloys studied. As a consequence, cobalt-based alloy wear particles lead to a pronounced foreign body reaction with all signs of an immune response. This work completes a previous study describing such adverse tissue reaction to metallic wear particles [M. Boehler, F. Kanz, B. Schwartz, I. Steffan, A. Walter, H. Plenk Jr and K. Knahr, The Journal of Bone and Joint Surgery 84-B, 128-136 (2002*)*]*.*

It will thus be appreciated that there is a need to develop a method which can reduce or substantially eliminate the hard particles contaminating grit-blasted surfaces without affecting too much the overall topography because a combination of topographies in the micrometer and nanometer range has shown to influence significantly the biological performance of implantable titanium devices in orthopaedic medicine and in dentistry.

The object of the present invention is therefore to provide such a method of surface finishing a bone implant that produces a rough surface whilst reducing contamination caused by blasting media.

According to a first aspect of the present invention there is provided a method of surface finishing a bone implant as defined in claim 1.

This combination of steps fulfills the object of the invention. The pickling of the implant in the pickling solution loosens any partially embedded abrasive blasting particles that may be contaminating the surface of the implant. Those loosened abrasive particles as well as the firmly adhering abrasive blasting particles are then detached by the mechanical cleaning action. Hence, the pickling process should not be designed to clean the surface of the implant or to produce additional pitting of the surface of the implant, as in some prior art procedures, but only to unlock any partially embedded abrasive blasting particles. Similarly, the mechanical action also should not be designed to provide any additional roughening of the surface, all of which is carried out during the initial abrasive blasting, but only to remove the loosened and firmly adhering abrasive particles.

Preferably, the abrasive particles used to roughen the surface of the implant are ceramic and/or metal particles. If the ceramic particles are used, these preferably comprise at least one of oxide particles, nitride particles and carbide particles.

Preferably also, the abrasive particles are propelled in a gaseous or liquid blasting medium.

Preferably also, the step of roughening the surface of the implant produces a surface roughness ranging from 3 to 7 µm of Ra inclusive and from 20 to 70 µm of Rt inclusive.

Preferably also, the pickling solution comprises one or a mixture of: a mixture of ammonium bi-fluoride and nitric acid; ammonium fluoride - ammonium bi-fluoride in acid mixtures; hydrofluoric acid based mixtures; sodium fluoride in acid mixtures; ammonium bi-fluoride and ammonium acetate in water; hydrochloric acid based mixtures; mixture of sulphuric and hydrochloric acid; and at least one fluoride salt, at least one acid and water.

Advantageously, the step of pickling the surface-roughened implant takes place by immersion of the implant in a mixture of ammonium bi-fluoride ([NH₄)]HF₂, 50g of powder for 1 liter), nitric acid (65% HNO₃, ₄₀₀ ml for 1 liter), and water to make up the solution to 1 liter. Preferably, the pickling takes place at room temperature, preferably a temperature of 27°C ± 2°C, for 20 seconds.

Preferably also, the mechanical action comprises with the use of ultrasounds when the implant is immersed in a liquid medium after pickling. Alternatively, the mechanical action comprises the blasting of the implant with substantially non-abrasive or only slightly-abrasive particulate media. In this latter case, preferably the particulate media comprise at least one of dry-ice pellets, crystalline particles of water soluble material, and particles of bioactive blasting material. The blasting medium can be gaseous, for example filtered air or nitrogen, or liquid, for example water.

In cases where dry-ice pellets are used these preferably comprise carbon dioxide snow flakes. Preferably also, they have an average diameter of 3 mm and are propelled in compressed air at an average pressure of 11 bars. The rate of supply of dry-ice pellets into the compressed air is preferably substantially of the order of 100 kilograms per hour.

In cases where crystalline particles of water soluble material are used, these preferably comprise particles of at least one of a sugar, sodium chloride, sodium sulfate and a mixture of any of the aforesaid materials. Alternatively, where particles of bioactive blasting material are used these preferably comprise particles of calcium phosphate and/or calcium carbonate.

Preferably also, the bone implant is comprised of one of titanium, zirconium, niobium, tantalum, an alloy based on any of the aforesaid elements, medical grade stainless steel, and a cobalt-chromium alloy.

The present invention will now be described by way of example with reference to the accompanying drawings, in which:
Figs. 1 to 3 are a set of x60 magnified optical images of the surface of a hip-joint implant after surface finishing respectively using three different surface finishing methods including in Fig. 3 a method in accordance with the present invention;
Fig. 4 is a graph illustration showing the percentage contamination of the surface of an implant by abrasive particles after surface finishing using five different surface finishing methods including the three methods illustrated in Figs. 1 to 3 as measured by image analysis on Back-Scattered-Electrons (BSE) micrographs and by optical micrographs;
Fig. 5 is an image produced by back scattering electron imaging (BSE imaging) at a magnification of x100 showing contamination of the surface of an implant by abrasive particles after grit blasting; and
Fig. 6 is a similar image to that shown in Fig. 5 but at a magnification of x200 and showing contamination of the surface of a similar implant by abrasive particles after surface finishing using a method in accordance with the present invention;
Figs. 7 and 8 are graphs showing the roughness parameters Ra and Rt respectively of the surface of an implant after different surface treatment methods including methods in accordance with the present invention.

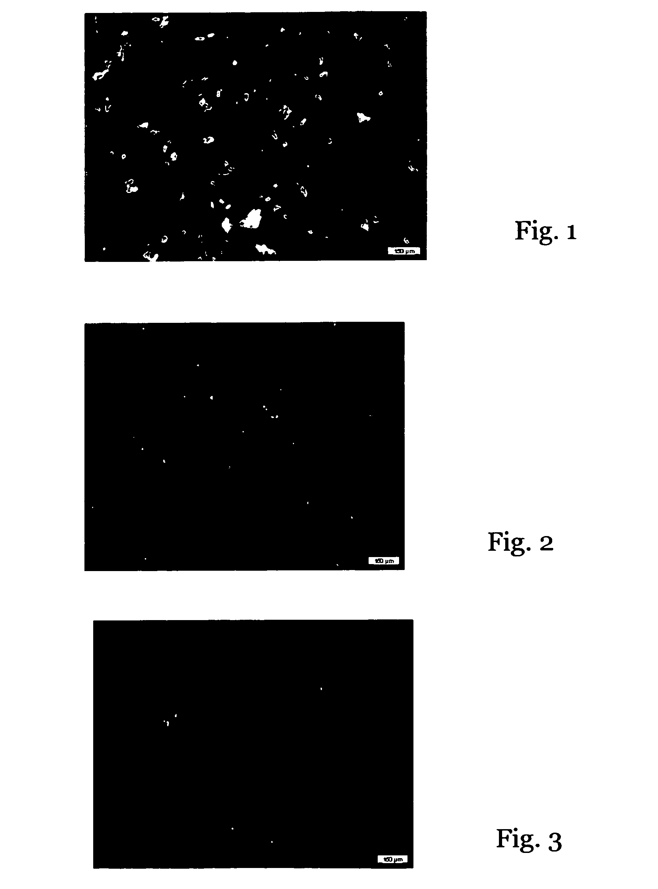

Figs. 1 to 3 respectively show the contamination of hip-joint implants by alumina particles after surface finishing using three different finishing methods. The hip-joint in each case was comprised of a Ti6Al7Nb alloy. In each image the contaminating alumina particles appear white or light grey in colour and the images were enhanced by a very short titanium etching applied for 20 seconds at 22 ± 2°C after each finishing treatment had been completed to obtain a better contrast with polarized light.

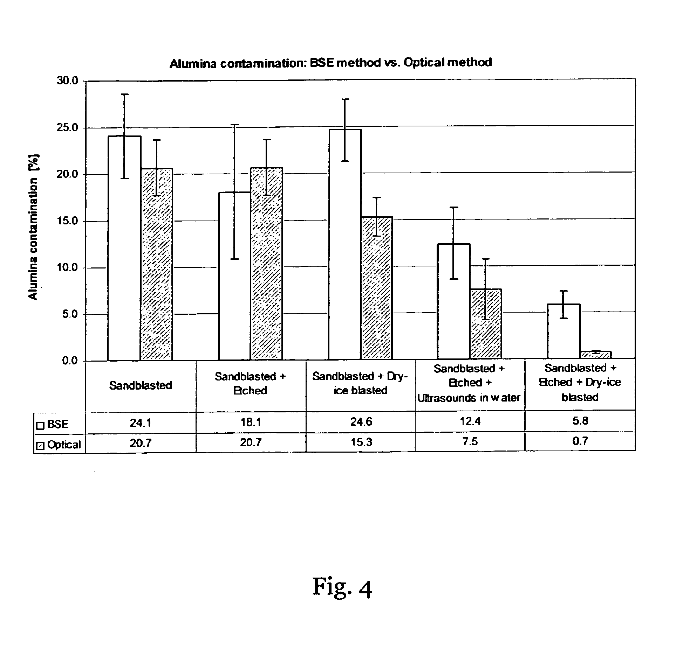

Fig. 4 shows graphically the mean percentage contamination of the surface of these implants obtained by image analysis on BSE micrographs and on optical micrographs after completion of five different surface finishing methods, including those used to produce Figs. 1 to 3.

To obtain the results from BSE micrographs the implant sample to be analyzed was introduced without any further treatment in a scanning electron microscope equipped with a BSE (Back-Scattered-Electron) detector. Five Back-Scattered-Electrons (BSE) micrographs were taken per sample at different randomly selected positions on the surface of the sample. The conditions used are the following: acceleration voltage: 20kV; spot-size: large (10); magnification: 100X; working distance: 25mm; detector adjustment: chemical contrast; amplification of the BSE signal: medium. Under such conditions, alumina appears black whereas the titanium surface appears white. An image analysis is then performed for each BSE micrograph with the contrast adjusted so that black corresponds to alumina. The sum of all the black areas compared to the entire surface of the micrograph analyzed gives the surface contamination in percent. The mean value as well as the standard deviation per sample are calculated from 5 different measurements performed on 5 different BSE micrographs. The accuracy of the method was controlled by Energy-Dispersive X-Ray (EDX) mapping performed in the same conditions (distance to detectors and magnification).

To obtain the results from optical micrographs the implant sample to be analyzed was chemically etched for 20 seconds at room temperature (22 ± 2°C) in a mixture of ammonium bifluoride ([NH4)]HF2, 50g of powder for 1 liter) and nitric acid (65% HNO_{3,} 400 ml for 1 liter) completed with water to obtain 1 liter of solution. The sample was then carefully rinsed with osmotic or purified water and left to dry in air. This enables a satisfactory optical contrast to be obtained. Six pictures were taken randomly of the surface of the implant using a digital camera coupled to a microscope equipped with a polarizing filter. The conditions used were the following: magnification of 60x, polarizing filter adjusted to obtain the maximum dark-field. Under such conditions, alumina appears white whereas the titanium surface appears black. An image analysis was then performed for each optical micrograph with the contrast adjusted so that white corresponds to alumina. The sum of all the white areas compared to the entire surface of the micrograph analyzed gives the surface contamination in percent. The mean value as well as the standard deviation per sample were then calculated from the 6 different measurements performed on the 6 different optical micrographs.

Fig. 1 shows the contamination of the surface after a hip-joint has been grit blasted using alumina-sand particles. This is a conventional surface treatment for such implants and, as can be seen from the image and by reference to the block labeled 'Sandblasted' in Fig. 4, the surface contamination is relatively high. Fig. 2 shows the contamination of the surface of a similar hip-joint after another conventional surface treatment wherein the hip-joint is first grit blasted using alumina particles and then 'cleaned' by dry-ice blasting directly on the grit blasted surface. Fig. 2 shows that the contamination is only slightly reduced by the ice-blasting treatment. If reference is made to the block labeled 'Sandblasted + Dry-ice blasted' in Fig. 4. it can also be seen that the dry-ice blasting produces no improvement on the alumina contamination when measured by BSE.

Fig. 4 also shows the result, of etching the grit-blasted surface (see block labelled 'Sandblasted + Etched'). Again, there is no significant improvement in surface contamination.

In contrast, Fig. 3 shows the results of a treating the surface of a similar hip-joint using a method also in accordance with the present invention wherein after grit-blasting using alumina sand the hip-joint was subjected to a short pickling treatment of around 20 seconds by immersion in a pickling solution prior to being dry-ice blasted. The addition of the short pickling step produces a significant improvement on the subsequent cleaning step by dry-ice blasting. As shown in Fig. 4, the alumina contamination is 76% lower than the original grit-blasted surface on the mean values when measured with BSE (on the mean values). Measured with the optical method, the reduction of contamination is of the order of 96%

The difference in magnification, in surface sensitivity and in precision between the two methods explains this discrepancy. The BSE method is very accurate and can measure very fine particles but it also measures, as deep as 3-5 microns underneath the surface, the alumina particles that are completely embedded in the substrate. On the other hand, the optical method is only sensitive to large alumina particles which are very slightly embedded.

In addition, it can be seen from Fig. 4 that the use of ultrasounds in water after etching (Sandblasted + Etched + Ultrasounds in water) reduces the contamination by around 48% when measured with BSE (on the mean values). This is not as effective as using dry-ice blasting but is still significantly better than prior art procedures.

Grit blasting is a stochastic process leading to a rough topography. The fact is that the coarser contributions to roughness often hide the fine surface roughness features when considering the standard "integral" roughness parameters such as Ra or Rt, which are defined according to ISO 4287-1997 and ASME B46.1-1995 such that Ra is arithmetic average of the absolute values of all points of the profile and Rt is the maximum peak-to-valley height of the entire measurement trace. These "integral" roughness parameters are scale dependent and depend also on the cutoff wavelength applied when measuring same. Typically, the roughness parameter Ra obtained after alumina grit blasting lies between 3 to 7 microns. However, it is known that such blasted surfaces are characterized by numerous surface roughness features ranging from the 100 microns scale to the nanometer scale.

In the present method, a bone implant made by a conventional method from a biocompatible material, such as any of titanium, zirconium, niobium, tantalum, an alloy based on any of the aforesaid elements, medical grade stainless steel, and a cobalt-chromium alloy is surface roughened. The roughening is produced by blasting with abrasive particles to produce micro and sub-micro topography. The blasting particles are preferably ceramic particles, such as oxides (for example: alumina, zirconia, titania, fused titanium dioxide, fused aluminium oxide), nitrides (for example carbon nitride, silicon nitride or boron nitride) or carbides (for example chromium carbide, silicon carbide or boron carbide), or metal particles. The medium use to propel the blasting particles can be gaseous, such as air or nitrogen (that may or may not have been dried), or liquid, for example water. After this blasting, the surface contamination by the blasting material has been typically found to be between 15 and 40% when measured by BSE and 10 to 30% when measured optically. The Ra and Rt roughness parameters are typically 3≤ Ra ≤7 µm and 20≤ Rt ≤70 µm respectively.

After surface blasting, the implant is subjected to a pickling process in a pickling or corrosive solution in order to loosen any partially embedded blasting particles from its surface. The exposure of the implant to the pickling solution must be carried out under controlled conditions for a controlled time in order to provide sufficient loosening of the partially-embedded blasting particles while minimizing topographical modifications to the surface of the implant. After pickling, the implant should be rinsed clean using osmotic or purified water and may be dried.

The pickling solution may comprise any of the following compositions:
- a mixture of ammonium bi-fluoride and nitric acid;
- ammonium fluoride - ammonium bi-fluoride in acid mixtures (such as hydrochloric acid, or sulphuric acid, or nitric acid);
- hydrofluoric acid based mixtures;
- sodium fluoride in acid mixtures (such as hydrochloric acid, and/or nitric acid);
- ammonium bi-fluoride and ammonium acetate in water;
- hydrochloric acid based mixtures;
- mixture of sulphuric and hydrochloric acid;
- at least one fluoride salt, at least one acid and water.
in the last case, the fluoride salt is preferably selected from a group comprising ammonium fluoride, ammonium bi-fluoride, potassium fluoride or sodium fluoride, or mixture thereof, the concentration of the fluoride salt being between 0.1 to 6 wt. % of the pickling solution. The acid is preferably selected from a group comprising nitric acid, hydrochloric acid, sulfuric acid, phosphoric acid, acetic acid, lactic acid, oxalic acid, tartaric acid, and mixtures thereof, the concentration of the acid in the pickling solution being about 0.1 to about 6N. The pickling solution may additionally comprise a chemically inert, water-soluble salt, selected from a group comprising sodium chloride, sodium sulfate, sodium bisulfate, sodium phosphate, sodium hydrogen phosphate, sodium dihydrogen phosphate, sodium nitrate, potassium chloride, potassium sulfate, potassium bisulfate, potassium hydrogen phosphate, potassium dihydrogen phosphate, potassium nitrate, ammonium sulfate, and mixtures thereof, the concentration of the sulfate salt being about 0.5 to 8 wt. % of the pickling solution.

Various controlled conditions may be applied during the pickling process. The temperature of the pickling bath can be between 5°C and boiling point, which may be above 100°C. The pickling bath may also be agitated. The agitation may be achieved by mechanical means or by bubbling an inert gas through the pickling solution. The pickling bath may also be aerated or kept under inert atmosphere, for example an argon or nitrogen atmosphere. The immersion time of the implant can be from a few seconds to several minutes depending on the aggressivity or etching rate of the pickling solution. The immersion time must be adjusted in order to keep the topographical parameters of the roughened implant surface before etching substantially the same. It has also been found that he efficacy of the pickling process can be enhanced electrochemically by anodic polarization of the implant in the bath.

After the pickling step, the method in accordance with the invention comprises a mechanical cleaning step in order to detach the blasting particles therefrom which have been loosened or loosened by the pickling process. Various forms of mechanical cleaning action including dry-ice blasting, as mentioned above, may be employed as follows:
- blasting of the surface of the implant using non- to slightly-abrasive particles at an appropriate pressure predetermined in order to avoid further roughening of the implant surface;
- ultrasonic cleaning of the implant in a liquid medium;
- liquid jetting of the implant;
- brushing the surface of the implant either manually or mechanically, for example with nylon brushes again in order to avoid further roughening of the implant surface.

Preferably, however, the surface of the implant is cleaned by blasting with a substantially non-abrasive particulate media such as one of the following:
- dry-ice pellets; for example made of compressed carbon dioxide snow flakes which have been extruded through a die. An alternative is to let liquid carbon dioxide flow directly through a special two-component concentric nozzle, the liquid expands as it exits and becomes a mixture of CO₂ snow (ice crystals) and gas which forms the core jet. In addition, compressed air (blasting medium) is fed in a ring shape;
- crystalline particles of water soluble material, such as particles of sugar(s), sodium chloride, sodium sulfate and the like, which can be easily removed from the implant surface, with or without the addition of flow/anti-caking agent(s);
- particles of bioactive blasting material, such as calcium phosphate or calcium carbonate, that are bioresorbable and to some extent water soluble but do not necessarily need to be removed from the surface.
The blasting medium can be gaseous, such as air, nitrogen or the like, which may be dried, or be liquid, such as water.

After the cleaning step, the surface contamination on the implant is further reduced but still present and is typically between 1% and 10% when measured by BSE and between 0.1% and 5% when measured optically. The Ra and Rt roughness parameters are typically 3≤ Ra ≥7 µm and 20≤ Rt ≤70 µm respectively, which are the same as after the blasting to produce the roughened surface. It will thus be appreciated that the processing conditions used in the pickling step and the cleaning step are adjusted in order to keep the topographical parameters approximately the same.

With a conventional implant such as a hip-joint comprised of a Ti-6AL-7NB alloy in accordance with ISO 5832-11, it has been found that a surface treatment using the following method produces optimal results.

First, the surface of the implant is subjected to an abrasive blasting procedure using a conventional alumina particulate (Al₂O₃, Biloxit Type K2O or K24). Dependent on the blasting apparatus used the pressure applied for blasting can range between 3 and 8 bars inclusive. Such a procedure produces a surface roughness ranging from 4 to 6 µm of Ra (average roughness, according to ISO 4287-1997 and ASME B46.1-1995). Next, the implant is pickled by immersion in a mixture of ammonium bi-fluoride ([NH₄)]HF₂, 50g of powder for 1 litre) and nitric acid (65% HNO₃, 400 ml for 1 litre) in water. The pickling bath should be maintained at a temperature of 27°C ± 2°C, i.e. approximately at room temperature. The immersion time of the implant in the bath should be for no more than 20 seconds. Finally, the implant is subjected to dry-ice blasting wherein it is blasted directly using 3 mm (average diameter) CO₂ pellets at an average of 11 bars with compressed air, using a dry-ice pellet supply of 100 kilograms per hour.

It has been found that the pickling step produces a maximum reduction of 40% of the alumina contamination (when considering mean values of contamination measured by the BSE method, and compared to a grit blasted surface). A mean reduction of 25% is measured on Ti-6Al-7Nb. The etching depth measured after 20 seconds of etching at 27°C on polished Ti-6Al-7Nb samples corresponds to 2.7 µm for cp Ti (Grade 2) and to 1.4 µm for Ti-6Al-4V (Grade 5, ELI) when measured with an optical profilometer FRT-MicroProf, equipped with a chromatic sensor CWL 0.3mm using the following method:
1. polishing the samples to a mirror finish, with a maximum Ra of 0.1 µm (finish N₃ or lower);
2. protecting the surface (with a coating resistant to the acid mixture) in order to leave a free line approximately 2 mm in width;
3. immersing the sample in the pickling bath for the desired time, at the desired temperature to pickle the unprotected zone;
4. removing the coating;
5. measuring the depth of the etched groove with a Laser or an optical profilometer by
   - mapping 4 x 4 mm ;
   - using a minimum point density of 80 pts/mm;
   - taking single profiles across the groove including both ridges in order to define the base line;
   - measuring the mean depth of the groove (on a minimum of 8 different profiles).

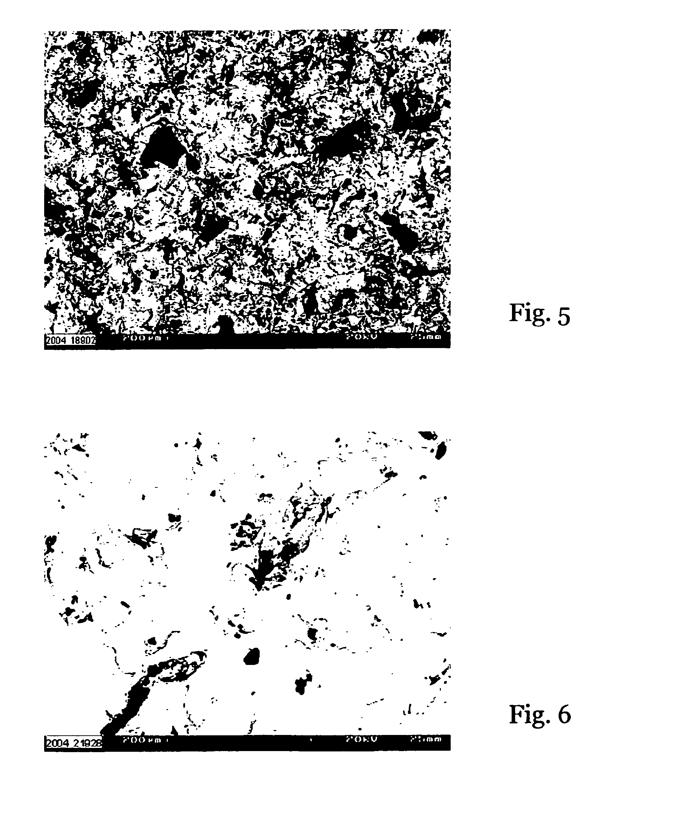

The improvement of the results over conventional surface treatment methods can be appreciated by a comparison of Figs. 5 and 6, which are both BSE images showing the contamination of the surface of an implant by abrasive particles. Fig. 5, which is at a magnification of X100, shows the surface contamination after a conventional grit blasting treatment using alumina particles whereas Fig. 6, which is a similar image but at a magnification of X200, shows the surface contamination after surface finishing using the aforesaid method in accordance with the present invention. In both cases the alumina particles appear black or dark grey and it is clear that the surface contamination in Fig. 6 where a method in accordance with the invention has been employed is significantly lower than that in Fig. 5.

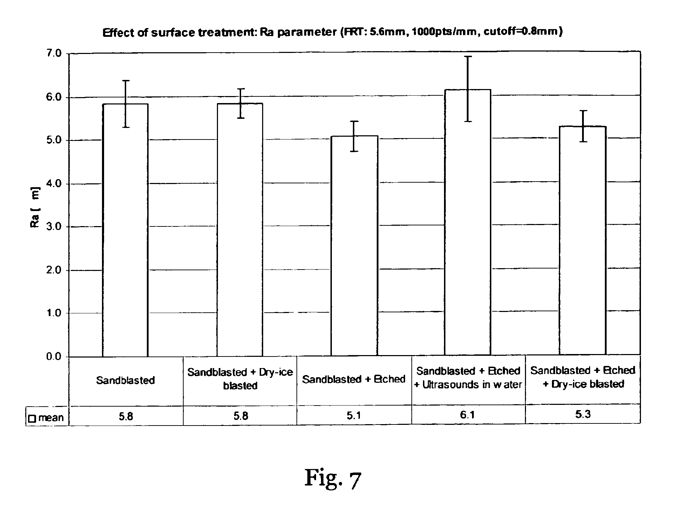

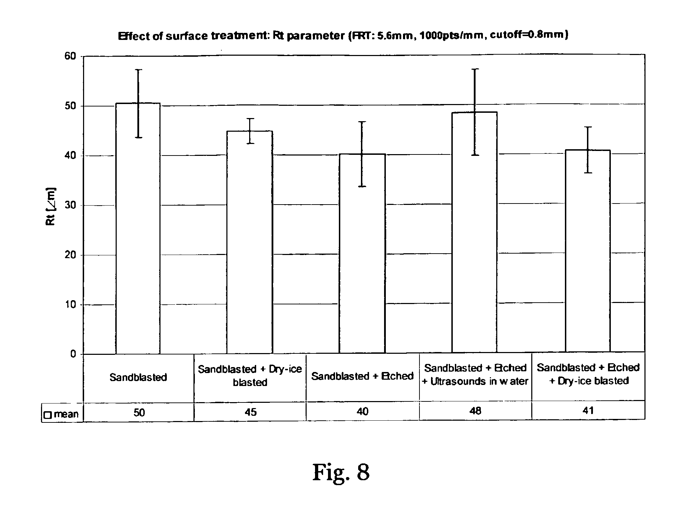

As previously stated, the pickling process used in the method in accordance with the invention is not designed to clean or structure the surface of the implant significantly with regard to the values of the alumina contamination as well as the roughness parameters before and after the pickling process. Rather, the pickling process is designed to loosen any partially embedded blasting particles. Also, the mechanical cleaning step is not designed to induce any additional roughening of the treated surface with regard to the roughness parameters before and after the combined process but to detach the abrasive particles from the surface which have been loosened by the pickling process. Hence, the abrasive blasting procedure used initially should be designed to produce the degree of surface roughness required in accordance with the type of implant in question and its proposed use. This can be appreciated by a consideration of the roughness parameters shown in tabular form in the following Table 1. Here, the roughness parameters of the surface of an implant after different surface treatments are shown having been measured with a non-contact optical profilometer FRT-MicroProf, equipped with a chromatic sensor CWL 0.3mm (length of measurement = 5.6 mm; 1000 pts/mm; cutoff = 0.8 mm, 0.8 mm was ignored at the beginning and the end of the profile; the calculation was performed using a Gaussian filter and an attenuation factor of 50% at the cut-off wavelength). Mean values and standard deviations (STDEV) are calculated from 6 measurements. The results for Ra and Rt are also shown graphically in Figs. 7 and 8 respectively. These roughness parameters have stayed staying within the same range for the various surface finishing methods used.

Table 1 is printed on the following sheet.

**Table 1**

| | Sandblasted | | Sandblasted + Dry-ice blasted | | Sandblasted + Etched | | Sandblasted + Etched + Ultrasounds in water | | Sandblasted + Etched + Dry-ice blasted | |
|---|---|---|---|---|---|---|---|---|---|---|
| | Mean | *STDEV* | Mean | *STDEV* | Mean | *STDEV* | Mean | *SDTEV* | Mean | *STDEV* |
| Ra [µm] | 5.8 | 0.5 | 5.8 | 0.3 | 5.1 | 0.3 | 6.1 | *0.8* | 5.3 | *0.4* |
| Rq [µm] | 7.4 | 0.7 | 7.3 | 0.5 | 6.4 | 0.6 | 7.7 | *0.9* | 6.7 | *0.5* |
| Rt [µm] | 50.5 | 6.8 | 44.8 | 2.5 | 40.2 | 6.5 | 48.5 | *8.6* | 40.8 | *4.8* |
| Rz(DIN)[µm] | 37.1 | 2.8 | 35.9 | 1.9 | 30.7 | 2.7 | 38.5 | *4.5* | 31.6 | *2.8* |
| Rmax [µm] | 45.9 | 9.2 | 42.8 | 3.8 | 38.2 | 6.3 | 46.8 | *9.2* | 39.1 | *6.2* |
| Rsk[-] | 0.0 | 0.2 | -0.1 | 0.2 | 01 | 0.2 | 0.0 | *0.3* | -0.2 | *0.2* |

The different roughness parameters are defined according to ISO 4287-1997 and ASME 846.1-1095:
Ra =arithmetic average of the absolute values of all points of the profile;
Rq =root mean square (RMS) of the values of all points of the profile,
Rt =maximum peak-to-valley height of the entire measurement trace,
Rz(DIN) = arithmetic average of the maximum peak-to-valley height of the roughness values of 5 consecutive sampling sections over the filtered profile;
Rmax = maximum individual roughness depth;
Rsk = amplitude distribution skew

## Claims

1. A method of surface finishing a bone implant comprising the steps of:
roughening a surface of the implant by blasting with abrasive particles;
pickling the surface-roughened implant in a pickling solution;
cleaning the roughened surface of the implant by mechanical action to detach the loosened blasting particles therefrom;
wherein the pickling step loosens any partially embedded abrasive blasting particles that may be contaminating the surface of the implant, by etching the surface of the implant to unlock the partially embedded abrasive blasting particles; **characterised in that** the pickling step leaves the surface of the implant with substantially the same roughness as is generated by the blasting with abrasive particles.

2. A method as claimed in claim 1, **characterized in that** the abrasive particles used to roughen the surface of the implant are ceramic particles.

3. A method as claimed in claim 2, **characterized in that** the ceramic abrasive particles comprise at least one of oxide particles, nitride particles and carbide particles.

4. A method as claim on any of claims 1 to 3, **characterized in that** the abrasive particles are propelled in a gaseous or liquid blasting medium.

5. A method as claimed in any of claims 1 to 4, **characterized in that** the step of roughening the surface of the implant produces a surface roughness ranging from 3 to 7 µm of Ra inclusive.

6. A method as claimed in any of claims 1 to 5, **characterized in that** the step of roughening the surface of the implant produces a surface roughness ranging from 20 to 70 µm of Rt inclusive.

7. A method as claimed in any of claims 1 to 6, **characterized in that** the pickling solution comprises one or a mixtures of: a mixture of ammonium bi-fluoride and nitric acid; ammonium fluoride - ammonium bi-fluoride in acid mixtures; hydrofluoric acid based mixtures; sodium fluoride in acid mixtures; ammonium bi-fluoride and ammonium acetate in water; hydrochloric acid based mixtures; mixture of sulphuric and hydrochloric acid; and at least one fluoride salt, at least one acid and water.

8. A method as claimed in any of claims 1 to 7, **characterized in that** the step of pickling the surface-roughened implant takes place by immersion of the implant in a mixture of ammonium bi-fluoride ([NH₄)]HF₂, 50g of powder for 1 liter), nitric acid (65% HNO₃, 400ml for 1 liter) and water to make up the solution to 1 liter.

9. A method as claimed in claim 8, **characterized in that** the implant is immersed in the mixture of ammonium bi-fluoride, nitric acid and water at room temperature for 20 seconds.

10. A method as claimed in claim 8 or claim 9, **characterized in that** the mixture of ammonium bi-fluoride, nitric acid and water is maintained at a temperature of 27°C ± 2°C.

11. A method as claimed in any of claims 1 to 10, **characterized in that** the mechanical action comprises the blasting of the implant with a substantially non-abrasive particulate media.

12. A method as claimed in claim 11, **characterized in that** the substantially non-abrasive particulate media are propelled in a gaseous or liquid blasting medium.

13. A method as claimed in claim 11 or claim 12 **characterized in that** the substantially non-abrasive particulate media comprise at least one of dry-ice pellets, crystalline particles of water soluble material and particles of bioactive blasting material.

14. A method as claimed in claim 13, **characterized** that the dry-ice pellets comprise carbon dioxide snow flakes.

15. A method as claimed in claim 13 or claim 14, **characterized in that** the dry-ice pellets have an average diameter of 3 mm and are propelled in compressed air at an average pressure of 11 bars.

16. A method as claimed in claim 15, **characterized in that** the rate of supply of dry-ice pellets into the compressed air is substantially of the order of 100 kilograms per hour.

17. A method as claimed in claim 13, **characterized in that** the crystalline particles of water soluble material comprise particles of at least one of a sugar, sodium chloride, sodium sulfate and a mixture of any of the aforesaid materials.

18. A method as claimed in claim 13, **characterized in that** the particles of bioactive blasting material comprise particles of calcium phosphate and/or calcium carbonate.

19. A method as claimed in any of claims 1 to 18, **characterized in that** the bone implant of comprised of one of titanium, zirconium, niobium, tantalum, an alloy based on any one of the aforesaid elements, medical grade stainless steel, and a cobalt-chromium alloy.

## Patentansprüche

1. Verfahren zur Oberflächenendbehandlung eines Knochenimplantats, das die folgenden Schritte umfasst:
Aufrauen einer Oberfläche des Implantats durch Bestrahlen mit abrasiven Partikeln;
Ätzen des oberflächenaufgerauten Implantats in einer Ätzlösung;
Reinigen der aufgerauten Oberfläche des Implantats durch mechanische Einwirkung, um die gelockerten Bestrahlungspartikel davon zu entfernen;
wobei der Ätzschritt irgendwelche teilweise eingebetteten Bestrahlungspartikel, welche die Oberfläche des Implantats kontaminieren könnten, durch Ätzen der Oberfläche des Implantats lockern, um die teilweise eingebetteten abrasiven Bestrahlungspartikel freizusetzen;
**dadurch gekennzeichnet, dass**
der Ätzschritt die Oberfläche des Implantats mit im Wesentlichen derselben Rauigkeit zurücklässt, wie sie durch das Bestrahlen mit abrasiven Partikeln erzeugt wird.

2. Verfahren nach Anspruch 1, **dadurch gekennzeichnet, dass** die abrasiven Partikel, die zum Aufrauen der Oberfläche des Implantats verwendet werden, Keramikpartikel sind.

3. Verfahren nach Anspruch 2, **dadurch gekennzeichnet, dass** die abrasiven Keramikpartikel Oxidpartikel, Nitridpartikel und/oder Carbidpartikel umfassen.

4. Verfahren nach einem der Ansprüche 1 bis 3, **dadurch gekennzeichnet, dass** die abrasiven Partikel in einem gasförmigen oder flüssigen Bestrahlungsmedium vorwärts getrieben werden.

5. Verfahren nach einem der Ansprüche 1 bis 4, **dadurch gekennzeichnet, dass** der Schritt des Aufrauens der Oberfläche des Implantats eine Oberflächenrauigkeit erzeugt, die im arithmetischen Mittel (R*a*) von 3 bis einschließlich 7 µm reicht.

6. Verfahren nach einem der Ansprüche 1 bis 5, **dadurch gekennzeichnet, dass** der Schritt des Aufrauens der Oberfläche des Implantats eine Oberflächenrauigkeit erzeugt, die in der Rautiefe (R*t*) von 20 bis einschließlich 70 µm reicht.

7. Verfahren nach einem der Ansprüche 1 bis 6, **dadurch gekennzeichnet, dass** die Ätzlösung ein Element aus der folgenden Aufzählung oder ein Gemisch umfasst aus: einem Gemisch aus Ammoniumbifluorid und Salpetersäure; Ammoniumfluorid - Ammoniumbifluorid in Säuregemischen; Gemischen auf Fluorwasserstoffsäurebasis; Natriumfluorid in Säuregemischen; Ammoniumbifluorid und Ammoniumacetat in Wasser; Gemischen auf Salzsäurebasis; Gemischen aus Schwefel- und Salzsäure; und mindestens ein Fluoridsalz, mindestens eine Säure und Wasser.

8. Verfahren nach einem der Ansprüche 1 bis 7, **dadurch gekennzeichnet, dass** der Schritt des Ätzens des oberflächenaufgerauten Implantats durch Eintauchen des Implantants in ein Gemisch aus Ammoniumbifluorid ([NH_{4]}HF₂, 50 g Pulver für 1 Liter), Salpetersäure (65% HNO₃, 400 ml für 1 Liter) und Wasser erfolgt, um die Lösung auf 1 Liter aufzufüllen.

9. Verfahren nach Anspruch 8, **dadurch gekennzeichnet, dass** das Implantat bei Raumtemperatur 20 Sekunden lang in das Gemisch aus Ammoniumbifluorid, Salpetersäure und Wasser eingetaucht wird.

10. Verfahren nach Anspruch 8 oder Anspruch 9, **dadurch gekennzeichnet, dass** das Gemisch aus Ammoniumbifluorid, Salpetersäure und Wasser auf einer Temperatur von 27°C ± 2°C gehalten wird.

11. Verfahren nach einem der Ansprüche 1 bis 10, **dadurch gekennzeichnet, dass** die mechanische Einwirkung das Bestrahlen des Implantats mit einem im Wesentlichen nicht abrasiven Feststoffteilchenmittel umfasst.

12. Verfahren nach Anspruch 11, **dadurch gekennzeichnet, dass** das im Wesentlichen nicht abrasive Feststoffteilchenmittel in einem gasförmigen oder flüssigen Bestrahlungsmittel vorwärts getrieben wird.

13. Verfahren nach Anspruch 11 oder Anspruch 12, **dadurch gekennzeichnet, dass** das im Wesentlichen nicht abrasive Feststoffteilchenmittel Trockeneis-Pellets, kristalline Partikel aus wasserlöslichem Material und/oder Partikel aus bioaktivem Bestrahlungsmaterial umfassen.

14. Verfahren nach Anspruch 13, **dadurch gekennzeichnet, dass** die Trockeneis-Pellets Kohlendioxidschneeflocken umfassen.

15. Verfahren nach Anspruch 13 oder Anspruch 14, **dadurch gekennzeichnet, dass** die Trockeneis-Pellets einen durchschnittlichen Durchmesser von 3 mm haben und in Druckluft mit einem durchschnittlichen Druck von 11 bar vorwärts getrieben werden.

16. Verfahren nach Anspruch 15, **dadurch gekennzeichnet, dass** die Zufuhrrate von Trockeneis-Pellets in die Druckluft im Wesentlichen ca. 100 Kilogramm pro Stunde beträgt.

17. Verfahren nach Anspruch 13, **dadurch gekennzeichnet, dass** die kristallinen Partikel aus wasserlöslichem Material Partikel aus Zucker, Natriumchlorid, Natriumsulfat und/oder ein Gemisch aus den vorgenannten Stoffen umfassen.

18. Verfahren nach Anspruch 13, **dadurch gekennzeichnet, dass** die Partikel aus bioaktivem Bestrahlungsmaterial Partikel aus Calziumphosphat und/oder Calziumcarbonat umfassen.

19. Verfahren nach einem der Ansprüche 1 bis 18, **dadurch gekennzeichnet, dass** das Knochenimplantat aus Titan, Zirkon, Niob, Tantal, einer Legierung auf Basis eines der vorgenannten Elemente, rostfreiem Stahl medizinischer Güte oder einer Kobalt-/Chromlegierung besteht.

## Revendications

1. Procédé de finition de surface d'un implant osseux comportant les étapes consistant à :
granuler une surface de l'implant par sablage à l'aide de particules abrasives,
décaper l'implant à surface granulée dans une solution de décapage,
nettoyer la surface granulée de l'implant par action mécanique pour détacher de celle-ci les particules de sablage relâchées,
dans lequel l'étape de décapage libère de quelconques particules de sablage abrasives partiellement incrustées qui peuvent contaminer la surface de l'implant, en gravant la surface de l'implant pour déverrouiller les particules de sablage abrasives partiellement incrustées, **caractérisé en ce que** l'étape de décapage laisse la surface de l'implant avec sensiblement la même rugosité que celle générée par le sablage à l'aide de particules abrasives.

2. Procédé selon la revendication 1, **caractérisé en ce que** les particules abrasives utilisées pour granuler la surface de l'implant sont des particules de céramique.

3. Procédé selon la revendication 2, **caractérisé en ce que** les particules abrasives de céramique comportent au moins l'une parmi des particules d'oxyde, des particules de nitrure et des particules de carbure.

4. Procédé selon l'une quelconque des revendications 1 à 3, **caractérisé en ce que** les particules abrasives sont propulsées dans un milieu de sablage gazeux ou liquide.

5. Procédé selon l'une quelconque des revendications 1 à 4, **caractérisé en ce que** l'étape de granulation de la surface de l'implant produit une granularité de surface dans la plage de 3 à 7 µm de Ra inclus.

6. Procédé selon l'une quelconque des revendications 1 à 5, **caractérisé en ce que** l'étape de granulation de la surface de l'implant produit une granularité de surface dans la plage de 20 à 70 µm de Rt inclus.

7. Procédé selon l'une quelconque des revendications 1 à 6, **caractérisé en ce que** la solution de décapage comporte un ou un mélange parmi : un mélange de bifluorure d'ammonium et d'acide nitrique, du fluorure d'ammonium-bifluorure d'ammonium dans des mélanges acides, des mélanges à base d'acide fluorhydrique, du fluorure de sodium dans des mélanges acides, du bifluorure d'ammonium et acétate d'ammonium dans de l'eau, des mélanges à base d'acide chlorhydrique, un mélange d'acide sulfurique et chlorhydrique, et au moins un sel de fluorure, au moins un acide et de l'eau.

8. Procédé selon l'une quelconque des revendications 1 à 7, **caractérisé en ce que** l'étape de décapage de l'implant à surface granulée a lieu par immersion de l'implant dans un mélange de bifluorure d'ammonium ([NH_{4]})HF₂, 50 g de poudre pour 1 litre), d'acide nitrique (HNO₃ à 65 %, 400 ml pour 1 litre) et d'eau pour amener la solution à 1 litre.

9. Procédé selon la revendication 8, **caractérisé en ce que** l'implant est immergé dans le mélange de bifluorure d'ammonium, d'acide nitrique et d'eau à température ambiante pendant 20 secondes.

10. Procédé selon la revendication 8 ou la revendication 9, **caractérisé en ce que** le mélange de bifluorure d'ammonium, d'acide nitrique et d'eau est maintenu à une température de 27 °C ± 2 °C.

11. Procédé selon l'une quelconque des revendications 1 à 10, **caractérisé en ce que** l'action mécanique comprend le sablage de l'implant à l'aide de milieux particulaires sensiblement non-abrasifs.

12. Procédé selon la revendication 11, **caractérisé en ce que** les milieux particulaires sensiblement non-abrasifs sont propulsés dans un milieu de sablage gazeux ou liquide.

13. Procédé selon la revendication 11 ou la revendication 12, **caractérisé en ce que** les milieux particulaires sensiblement non-abrasifs comportent au moins l'une parmi des pastilles de glace sèche, des particules cristallines de matériau soluble dans l'eau et des particules de matériau de sablage bioactif.

14. Procédé selon la revendication 13, **caractérisé en ce que** les pastilles de glace sèche comportent des flocons de neige carbonique.

15. Procédé selon la revendication 13 ou la revendication 14, **caractérisé en ce que** les pastilles de glace sèche on un diamètre moyen de 3 mm et sont propulsées dans de l'air comprimé à une pression moyenne de 11 bars.

16. Procédé selon la revendication 15, **caractérisé en ce que** la vitesse d'alimentation des pastilles de glace sèche dans l'air comprimé est sensiblement de l'ordre de 100 kilogrammes par heure.

17. Procédé selon la revendication 13, **caractérisé en ce que** les particules cristallines de matériau soluble dans l'eau comportent des particules d'au moins l'un parmi un sucre, chlorure de sodium, sulfate de sodium et un mélange de l'un quelconque des matériaux mentionnés ci-dessus.

18. Procédé selon la revendication 13, **caractérisé en ce que** les particules de matériau de sablage bioactif comportent des particules de phosphate de calcium et/ou de carbonate de calcium.

19. Procédé selon l'une quelconque des revendications 1 à 18, **caractérisé en ce que** l'implant osseux est constitué de l'un parmi du titane, zirconium, niobium, tantale, un alliage basé sur l'un quelconque des éléments mentionnés ci-dessus, acier inoxydable de qualité médicale, et un alliage de cobalt-chrome.
